(19) Europäisches Patentamt
European Patent Office
Office européen des brevets

(11) Publication number: **0 643 966 A1**

(12) **EUROPEAN PATENT APPLICATION published in accordance with Art. 158(3) EPC**

(21) Application number: **94907663.2**

(22) Date of filing: **23.02.94**

(86) International application number: **PCT/JP94/00275**

(87) International publication number: **WO 94/20106 (15.09.94 94/21)**

(51) Int. Cl.[6]: **A61K 31/475**, C07D 498/22

(30) Priority: **03.03.93 JP 42473/93**

(43) Date of publication of application: **22.03.95 Bulletin 95/12**

(84) Designated Contracting States: **DE FR GB IT**

(71) Applicant: **KYOWA HAKKO KOGYO KABUSHIKI KAISHA**
**6-1, Ohte-machi 1-chome**
**Chiyoda-ku**
**Tokyo 100 (JP)**

(72) Inventor: **MURAKATA, Chikara**
**2-11-2-702, Bessho**
**Hachioji-shi,**
**Tokyo 192-03 (JP)**
Inventor: **SAITHO, Yutaka**
**3-9-13, Naka-machi**
**Machida-shi,**
**Tokyo 194 (JP)**
Inventor: **AKINAGA, Shiro**
**1188, Shimotogari**
**Nagaizumi-cho**
**Sunto-gun,**
**Shizuoka 411 (JP)**
Inventor: **OKABE, Masami**
**30-5, Kamo**
**Mishima-shi,**
**Shizuoka 411 (JP)**

(74) Representative: **Casalonga, Axel et al**
**BUREAU D.A. CASALONGA - JOSSE**
**Morassistrasse 8**
**D-80469 München (DE)**

(54) **SENSITIVITY ENHANCER FOR ANTINEOPLASTIC AGENT.**

(57) The invention provides a sensitivity enhancer for antineoplastic agents which contains as the active ingredient an indolocarbazole derivative represented by general formula (I) or a pharmacologically acceptable salt thereof, wherein $R^1$ represents hydrogen, lower alkyl or lower alkanoyl; A represents a group represented by general formula (1) (wherein $R^2$ represents hydrogen, lower alkyl or lower alkanoyl, and $R^3$ represents hydrogen or lower alkyl) when one of $W^1$ and $W^2$ represents hydrogen and the other thereof represents hydrogen or hydroxy, or alternatively $W^1$ and $W^2$ are combined together to represent oxygen, excluding the case where all of the $R^1$, $R^2$, $W^1$ and $W^2$ are hydrogen atoms and $R^3$ is methyl; or A represents a group represented by general formula (2) [wherein X represents a single bond or -CO-; m represents an integer of 0 to 6; and Y represents hydrogen, $OR^4$ (wherein $R^4$ represents hydrogen, lower alkyl, aralkyl or $CONR^5R^6$, wherein $R^5$ and $R^6$ represent each independently hydrogen or lower alkyl, or alternatively $R^5$ and $R^6$ are combined together to form a heterocyclic group together with the nitrogen atom to which they are bonded), $NR^7R^8$ (wherein $R^7$ and $R^8$ represent each independently hydrogen or lower alkyl, or alternatively $R^7$ and $R^8$ are combined together to form a heterocyclic group together with the nitrogen atom to which they are bonded), or (un)substituted aryl] when $R^1$ represents lower alkyl or lower alkanoyl and one of $W^1$ and $W^2$ represents hydrogen while the other thereof

EP 0 643 966 A1

represents hydrogen or hydroxy, or when $R^1$ represents hydrogen and one of $W^1$ and $W^2$ represents hydrogen while the other thereof represents hydroxy. The invention also provides another indolocarbazole derivative represented by general formula (II) or a pharmacologically acceptable salt thereof, wherein $R^{1a}$ represents lower alkyl or lower alkanoyl; W represents hydrogen or hydroxy; and $R^{4a}$ represents lower alkyl.

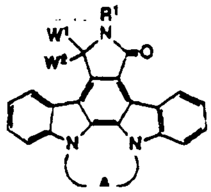

(I)

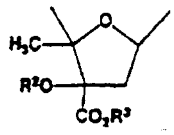

(1)

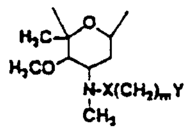

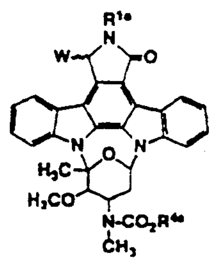

(2)

Field of the Invention

This invention relates to an agent for potentiating sensitivity to an antitumor agent.

Background of the Invention

Clinical use of antitumor agents in chemotherapy for cancers has involved the problem of resistance to the antitumor agents. Some kinds of cancers are known to acquire multiple drug resistance and become refractory to the actions of many antitumor agents. As the compounds which are effective to overcome multiple drug resistance, Verapamil [Yuki Gosei Kagaku, 49, 980(1991)] and the like, and indolocarbazole derivatives, such as NA381, NA382, and NA478 [Cancer Letters, 64, 177(1992); J. Pharm. Pharmacol., 45, 43(1993); and J. Antibiotics, 46, 353(1993)] are known.

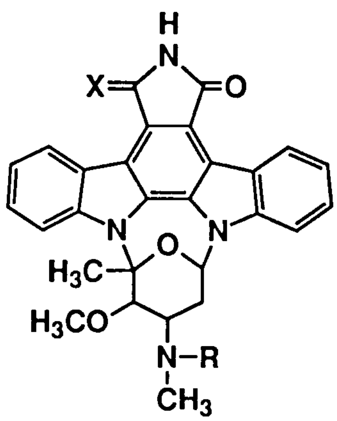

**NA381; X=H,H, R=$CO_2CH_2CH_3$**
**NA382; X=O, R=$CO_2CH_2CH_3$**
**NA478; X=H,H, R=$CO_2CH_2C_6H_5$**

Indolocarbazole derivatives having antitumor activity are also known (WO 89/07105; and Japanese Published Unexamined Patent Application Nos. 295588/88 and 295589/88).

Disclosure of the Invention

The present invention relates to an agent for potentiating sensitivity to an antitumor agent comprising, as an active ingredient, an indolocarbazole derivative represented by formula (I):

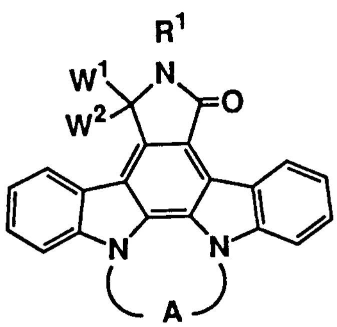

(I)

wherein
when $R^1$ represents hydrogen, lower alkyl, or lower alkanoyl, and one of $W^1$ and $W^2$ represents hydrogen with the other representing hydrogen or hydroxy, or alternatively $W^1$ and $W^2$ are combined to represent oxygen, then A represents

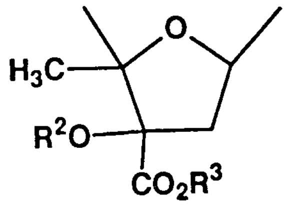

(wherein $R^2$ represents hydrogen, lower alkyl, or lower alkanoyl; and $R^3$ represents hydrogen or lower alkyl) with the proviso that when $R^1$, $R^2$, $W^1$, and $W^2$ are hydrogen, then $R^3$ is hydrogen or other lower alkyl than methyl;
when $R^1$ represents lower alkyl or lower alkanoyl, and one of $W^1$ and $W^2$ represents hydrogen with the other representing hydrogen or hydroxy, or when $R^1$ represents hydrogen, and one of $W^1$ and $W^2$ represents hydrogen with the other representing hydroxy, then A represents

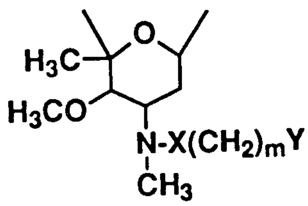

[wherein X represents a single bond or -CO-; m represents an integer of 0 to 6; and Y represents hydrogen, $OR^4$ (wherein $R^4$ represents hydrogen, lower alkyl, aralkyl, or $CONR^5R^6$, wherein $R^5$ and $R^6$ independently represent hydrogen or lower alkyl, or alternatively $R^5$ and $R^6$ are taken together with the nitrogen atom to form a heterocyclic group), $NR^7R^8$ (wherein $R^7$ and $R^8$ independently represent hydrogen or lower alkyl, or alternatively $R^7$ and $R^8$ are taken together with the nitrogen atom to form a heterocyclic group), or substituted or unsubstituted aryl;
or a pharmaceutically acceptable salt thereof.

The present invention further relates to a method for potentiating sensitivity to an antitumor agent comprising administration of an effective amount of an indolocarbazole derivative represented by formula (I) or a pharmaceutically acceptable salt thereof.

The present invention furthermore relates to the use of an indolocarbazole derivative represented by formula (I) or a pharmaceutically acceptable salt thereof for the preparation of a pharmaceutical composition useful for potentiation of sensitivity to an antitumor agent.

The present invention also provides an indolocarbazole derivative represented by formula (II):

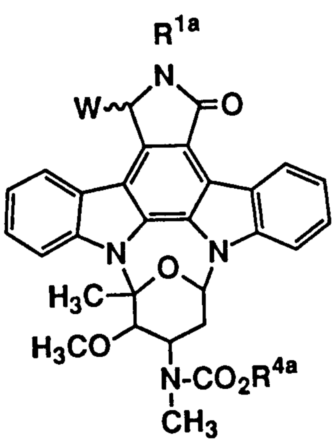

(II)

wherein $R^{1a}$ represents lower alkyl or lower alkanoyl; W represents hydrogen or hydroxy; and $R^{4a}$ represents lower alkyl;
or a pharmaceutically acceptable salt thereof.

The compounds represented by formula (I) and formula (II) are hereinafter referred to as Compound (I) and Compound (II), respectively. The same applies to the compounds of other formula numbers.

In the definitions of the groups in formula (I) and formula (II), the lower alkyl and the lower alkyl moiety of the lower alkanoyl mean a straight-chain or branched alkyl group having 1 to 6 carbon atoms, such as methyl, ethyl, propyl, isopropyl, butyl, sec-butyl, tert-butyl, pentyl, and hexyl. The aralkyl means an aralkyl group having 7 to 13 carbon atoms, such as benzyl, phenethyl, and naphthylmethyl. The heterocyclic group formed with the nitrogen atom means pyrrolidinyl, imidazolidinyl, pyrazolidinyl, piperidino, morpholino, piperazinyl, N-methylpiperazinyl, indolinyl, isoindolinyl, and the like.

The aryl means phenyl, naphthyl, and the like. The substituted aryl means the aryl which is independently substituted with 1 to 3 substituents, such as lower alkyl, lower alkoxy, amino, halogen, and nitro. In the definitions of the substituents, the lower alkyl and the lower alkyl moiety of the lower alkoxy have the same meanings as defined above, and the halogen means fluorine, chlorine, bromine, and iodine.

The pharmaceutically acceptable salt of Compound (I) includes pharmaceutically acceptable acid addition salts, pharmaceutically acceptable metal salts, pharmaceutically acceptable ammonium salts, pharmaceutically acceptable organic amine addition salts, and pharmaceutically acceptable amino acid addition salts.

Examples of the pharmaceutically acceptable acid addition slats of Compound (I) are inorganic acid addition salts, such as hydrochloride, sulfate, and phosphate, and organic acid addition salts, such as acetate, maleate, fumarate, tartrate, and citrate. Examples of the pharmaceutically acceptable metal salts are alkali metal salts, such as sodium salt and potassium salt, alkaline earth metal salts, such as magnesium salt and calcium salt, aluminum salt, and zinc salt. Examples of the pharmaceutically acceptable ammonium salts are ammonium salt and tetramethylammonium salt. Examples of the pharmaceutically acceptable organic amine addition salts are salts with morpholine and piperidine. Examples of the pharmaceutically acceptable amino acid addition salts are salts with lysine, glycine, and phenylalanine.

The antitumor agents to which an agent for potentiating sensitivity of the present invention is applicable include Adriamycin, Vincristine, Vinblastine, Daunomycin, Etoposide, Actinomycin, and Aclacinomycin.

The processes for producing Compounds (II) are described below.

Process 1:

Compound (II) can be prepared by the following reaction steps:

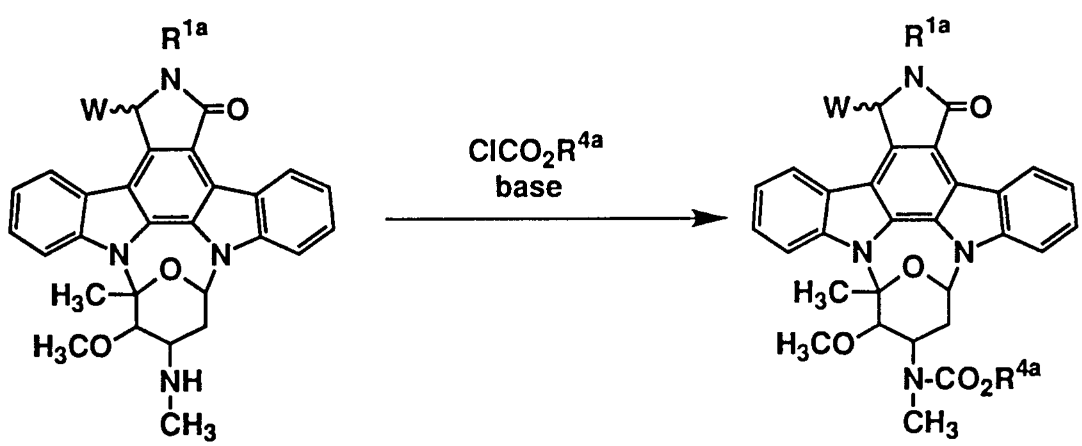


(III) (II)

(In the formulae, $R^{1a}$, W, and $R^{4a}$ have the same meanings as defined above.)

Compound (II) can be obtained by reaction of Compound (III) with a lower alkyl chloroformate (1 to 3 equivalents) in the presence of an appropriate base, such as sodium bicarbonate (3 to 10 equivalents) in a solvent, such as water-containing acetonitrile.

Process 2:

Compound (II-a), which is Compound (II) in which W is hydrogen, can also be prepared by the following reaction steps:

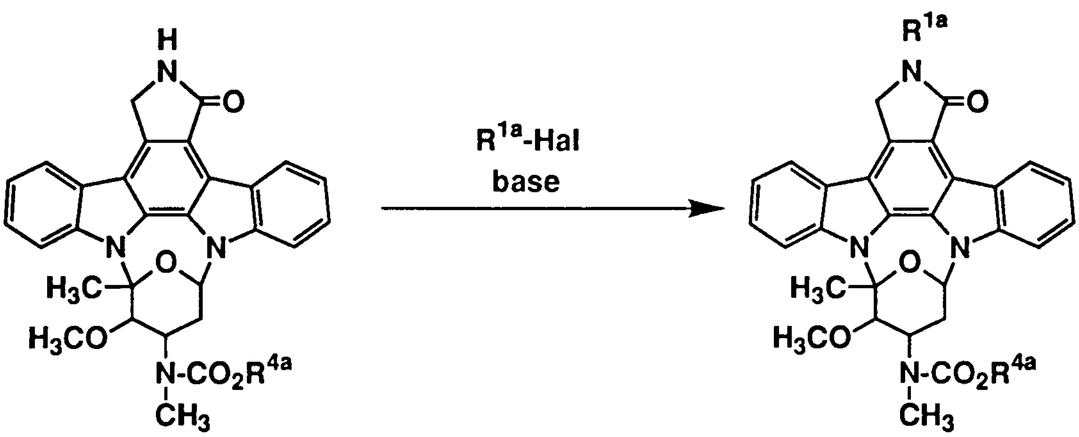


(IV) (II-a)

(In the formulae, Hal represents chlorine, bromine, or iodine; and $R^{1a}$ and $R^{4a}$ have the same meanings as defined above.)

Compound (II-a) can be obtained by reaction of Compound (IV) with a lower alkyl halide (1 to 5 equivalents) or a lower alkanoyl halide (1 to 5 equivalents) in the presence of an appropriate base, such as sodium hydride (1 to 2 equivalents), in a solvent, such as dimethylformamide.

The desired compounds in the above-described processes can be isolated and purified by an appropriate combination of the methods conventionally used in organic synthetic chemistry, for example, extraction, crystallization, and various kinds of chromatography.

In the case where a salt of Compound (II) is desired and it is produced in the form of the salt, it can be subjected to purification as such. In the case where Compound (II) is produced in the free state, it is dissolved or suspended in a suitable organic solvent, followed by addition of an acid or a base to form a salt.

Compounds (I) and pharmaceutically acceptable salts thereof may be in the form of adducts with water or various solvents, which can also be used as the therapeutic agents of the present invention.

Embodied examples of Compounds (I) and their physical data are shown in Tables 1 and 2, respectively.

## Table 1

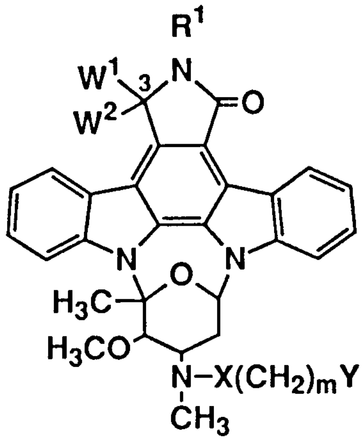

| Compd. No. | $R^1$ | $W^1$ | $W^2$ | $-X(CH_2)_mY$ | MS(m/z) |
|---|---|---|---|---|---|
| I-1 | $CH_3$ | H | H | -H | 480($M^+$+1) |
| I-2 | $CH_3$ | H | H | $-CO_2CH_2C_6H_5$ | 615($M^+$+1) |
| I-3 | H | H | OH | 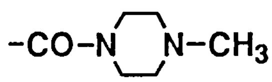 | 609($M^+$+1) |
| I-4 | H | H | OH | 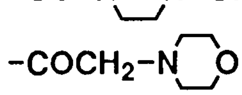 | 610($M^+$+1) |
| I-5 | H | H | OH | $-COCH_2N(CH_3)_2$ | 568($M^+$+1) |
| I-6 | H | H | OH | 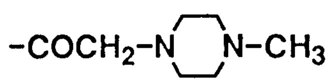 | 623($M^+$+1) |
| I-7 | H | H | OH | 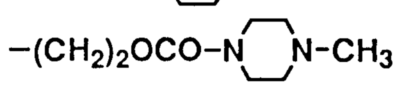 | 653($M^+$+1) |
| I-8 | H | H | OH | $-(CH_2)_2OH$ | 527($M^+$+1) |
| I-9 | H | OH | H | $-(CH_2)_2OH$ | 527($M^+$+1) |
| I-10 | $CH_3$ | H | H | $-CO_2CH_2CH_3$ | 553($M^+$+1) |

Compounds (I-3) to (I-7) are each an about 1:1 mixture of stereoisomers.

## Table 2

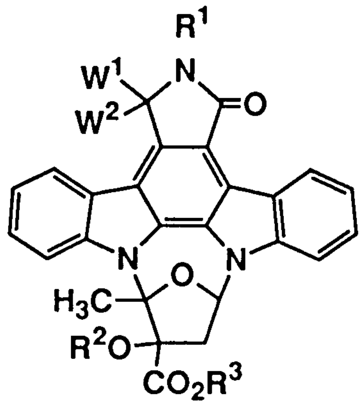

| Compd. No. | $R^1$ | $W^1$ | $W^2$ | $R^2$ | $-R^3$ | MS(m/z) |
|---|---|---|---|---|---|---|
| I-11 | H | H | H | H | $-CH_2CH_3$ | 481($M^+$+1) |
| I-12 | H | H | H | H | $-(CH_2)_2CH_3$ | 495($M^+$+1) |
| I-13 | H | H | H | H | $-(CH_2)_3CH_3$ | 509($M^+$+1) |
| I-14 | H | H | H | H | $-(CH_2)_5CH_3$ | –* |
| I-15 | H | =O | | H | $-(CH_2)_5CH_3$ | 551($M^+$+1) |
| I-16 | $CH_3$ | H | H | $CH_3$ | $-CH_3$ | 495($M^+$+1) |
| I-17 | $COCH_3$ | H | H | $COCH_3$ | $-CH_3$ | 551($M^+$+1) |

*; Melting point: 145–147.5C° (methanol)

The processes for preparation and more detailed physical data for Compounds (I-1) and (I-2) are described in WO 89/07105; those for Compounds (I-23) to (I-14) are described in Japanese Published Unexamined Patent Application No. 155284/87; those for Compound (I-15) are described in Japanese Published Unexamined Patent Application No. 295589/88; those for Compound (I-16) are described in Japanese Published Unexamined Patent Application No. 295588/88; and those for Compound (I-17) are described in J. Antibiotics, 38, 1437(1985). The processes for preparation and more detailed physical data for Compounds (I-3) to (I-9) and (I-10) are shown in Examples and Reference Examples.

Compounds (I) can be administered orally or parenterally in the dose form of tablets, granules, capsules, syrups, ointments, creams, injections, etc., prepared in a conventional manner, depending on the aim and method of administration. In the case of tablets, for example, tablets each containing 50 to 200 mg of the active ingredient are suitably used. In preparing tablets, vehicles (e.g., lactose, glucose, sucrose, mannitol, and methyl cellulose), disintegrators (e.g., starch, sodium alginate, calcium carboxymethyl cellulose, and crystalline cellulose), lubricants (e.g., magnesium stearate and talc), binders (e.g., gelatin, polyvinyl alcohol, polyvinyl pyrrolidone, hydroxypropyl cellulose, and methyl cellulose), surfactants (e.g., sucrose fatty acid esters and sorbitol fatty acid esters), and the like are employed in a conventional manner. In preparing granules, vehicles (e.g., lactose and sucrose), disintegrators (e.g., starch), binders (e.g., gelatin), and the like are used in a conventional manner. In preparing powders, vehicles (such as lactose and mannitol) are used in a conventional manner. In the case of capsules, capsules each containing 50 to 200 mg of the active ingredient are suitably used. In preparing capsules, gelatin, water, sucrose, gum arabic, sorbitol, glycerin, crystalline cellulose, magnesium stearate, talc, etc. are used in a conventional manner. In preparing syrups, sugars (e.g., sucrose), water, ethanol, etc. are used in a conventional manner. In preparing ointments, ointment bases (e.g., vaseline, liquid paraffin, lanolin, and Macrogol), emulsifiers

(e.g., sodium lauryl lactate, benzalkonium chloride, sorbitan monofatty acid esters, sodium carboxymethyl cellulose, and gum arabic), and the like are used in a conventional manner. In preparing injections, solvents (such as water, physiological saline, vegetable oils (e.g., olive oil and peanut oil), ethyl oleate, and propylene glycol), solubilizing agents (e.g., sodium benzoate, sodium salicylate, and urethane), isotonic agents (e.g., sodium chloride and glucose), preservatives (e.g., phenol, cresol, p-hydroxybenzoic acid esters, and chlorobutanol), antioxidants (e.g., ascorbic acid and sodium pyrosulfite), and the like are used in a conventional manner.

Compound (I) can be used as an adjuvant of an active ingredient possessing antitumor activity or alternatively Compound (I) may be separately administered from the administration of the antitumor agent. The dose of Compound (I) is 0.1 to 1000 times as much as that of an active ingredient possessing antitumor activity of the antitumor agent.

The toxicity and pharmacological activities of Compounds (I) are shown by the following Test Examples.

Test Example 1 Acute Toxicity Test

The test was conducted using three 6-week-old male BALB/c mice per group. A test Compound (I) was suspended in a 0.3% aqueous solution of carboxymethyl cellulose, and 0.1 ml of the suspension was intraperitoneally administered to each mouse per 10 g-body weight. A 50% lethal dose ($LD_{50}$) was obtained from the mortality 1 week after the administration. As a result, the $LD_{50}$ values of Compounds (I-1) to (I-17) were above 50 mg/kg.

Test Example 2 Test on Vindesine Resistance Using Adriamycin-Resistant Human Ovary Cancer Cells (A2780/ADM)

A2780/ADM cells were diluted with an RPMI 1640 medium (430-1800 EB, produced by GIBCO) containing 10% fetal bovine serum (200-6140AG, produced by GIBCO) and 1% glutamine (320-5030 PG, produced by GIBCO) to a concentration of 30000 cells/ml. One milliliter of the cell suspension was inoculated to each well of a 24-well microtiter plate and cultured under conditions of 37 °C, 5% $CO_2$, and a humidity of 95% or higher for 1 day. A mixture of 0.1 ml of a Vindesine solution diluted serially with the above medium and 0.1 ml of the above medium was added to each well as a control group. Separately, a mixture of a test compound (I) diluted to such a concentration at which the test compound alone exhibits 20 to 30% cytotoxicity and Vindesine (totalizing 0.2 ml) was added to each well as a test group. The cells in each well were further cultured under the same conditions as above for additional 3 days, and the number of the cells was counted with a Coulter counter to obtain a 50% growth inhibitory concentration ($IC_{50}$) of the control groups and the test groups. The results obtained are shown in Table 3 below. The compounds used in the present invention were proved to have a significant effect of overcoming the drug resistance.

Table 3

| Test Compound | $IC_{50}$ of Test Compound Alone (μM) | Concentration of Test Compound (μM) | $IC_{50}$ of Vindesine (μM) |
|---|---|---|---|
| - | - | - | 1.3 |
| I-1 | 0.26 | 0.1 | 0.51 |
| I-2 | >100 | 10 | 0.0009 |
| I-3 | 10.0 | 5.0 | 0.0036 |
| I-4 | 7.0 | 5.0 | 0.21 |
| I-5 | 9.1 | 5.0 | 0.27 |
| I-6 | 12.5 | 7.5 | 0.70 |
| I-7 | 9.5 | 5.0 | 0.047 |
| I-8 | 17.2 | 10 | 0.16 |
| I-9 | 9.2 | 3.0 | 0.38 |
| I-10 | >10 | 3.0 | 0.001 |
| I-11 | 0.16 | 0.05 | 0.77 |
| I-12 | 0.42 | 0.3 | 0.22 |
| I-13 | 1.1 | 0.3 | 0.31 |
| I-14 | 4.8 | 3.0 | 0.012 |
| I-15 | 2.8 | 1.0 | 0.28 |
| I-16 | 13 | 5.0 | 0.0007 |
| I-17 | >100 | 30 | 0.0013 |

Test Example 3 Test on Vincristine Resistance Using Vincristine-Resistant Mouse Leukemia Cells (P388/VCR)

Vincristine-resistant P388 cells (P388/VCR) were suspended in physiological saline, and $5x10^6$ cells/0.2 ml were intraperitoneally implanted to grouped 6-week-old $CDF_1$ strain male mice, each group consisting of 5 mice. A prescribed amount of Vincristine alone or a mixture of Vincristine and a test compound (I) was intraperitoneally administered for consecutive 5 days from the next day of the implantation. The effect of overcoming the drug resistance was evaluated in terms of T/C, a percentage of the number of survival days of a test group to the number of survival days of a control group.

Vincristine (VCR) used was Vincristine Sulfate, produced by Shionogi & Co., Ltd., dissolved in physiological saline. Compound (I) was suspended in a 0.3% aqueous solution of carboxymethyl cellulose and administered in an amount of 0.1 ml per 10 g-b.w. The results obtained are shown in Table 4. Compound (I-2) revealed excellent effects to overcome the drug resistance.

Table 4

| Compound | T/C (%) |
|---|---|
| Control (no treatment) | 100 |
| Compound (I-2); 22.2 mg/kg | 102 |
| VCR; 0.4 mg/kg | 121 |
| VCR; 0.2 mg/kg | 116 |
| VCR; 0.1 mg/kg | 112 |
| VCR; 0.1 mg/kg + Compound (I-2); 22.2 mg/kg | 152 |

Certain embodiments of the present invention are illustrated in the following Examples and Reference Examples.

## The Best Mode for Carrying out the Invention

### Example 1 Compound (I-10)

In 3 ml of dimethylformamide was dissolved 100 mg of Compound j obtained in Reference Example 7, and 11 mg of sodium hydride was added thereto at -23°C, followed by stirring for 20 minutes. To the mixture was added 0.035 ml of methyl iodide at the same temperature, followed by stirring at 0°C for 2 hours. The reaction mixture was diluted with water and chloroform, the organic layer was separated, washed with an aqueous solution of sodium chloride, and dried over anhydrous sodium sulfate, and the solvent was removed by distillation. The residue was purified by preparative thin layer chromatography to give 78 mg of Compound (I-10).

$^1$H-NMR ($CDCl_3$) δ (ppm); 2.469 (s, 3H), 2.545 (s, 3H), 2.781 (s, 3H), 3.406 (s, 3H), 4.973 (s, 2H), 6.767 (dd, 1H, J=5.5, 8.6Hz), 7.248-7.976 (m, 7H), 9.512 (d, 1H, J=7.9Hz)

### Example 2 Tablets

Tablets having the following composition were prepared in a conventional manner.

| Formulation: | |
|---|---|
| Compound (I-2) | 168 mg |
| Lactose | 84 mg |
| Corn starch | 40.2 mg |
| Polyvinyl alcohol | 4 mg |
| Avicel | 2.8 mg |
| Magnesium stearate | 1 mg |
| | 300 mg |

### Example 3 Capsules

Capsules having the following composition were prepared in a conventional manner.

Compound (I-3) (200 mg) was mixed with 5 mg of talc, 165 mg of lactose, and 20 mg of corn starch. The mixture was passed through a 0.5 mm mesh sieve and charged in gelatin capsules of a desired size to obtain capsules.

Reference Examples are described below. The structure of Compounds a to i in the Reference Examples are shown in Table 5.

## Table 5

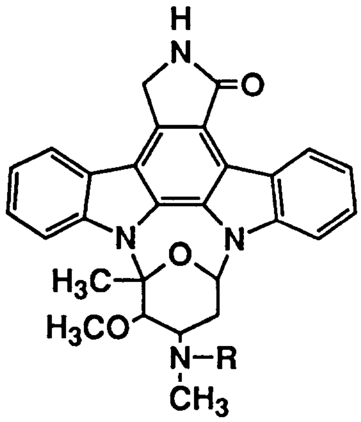

| Ref. Ex. No. | Compd. No. | −R |
|---|---|---|
| 1 | a | $-(CH_2)_2OH$ |
| 2 | b | 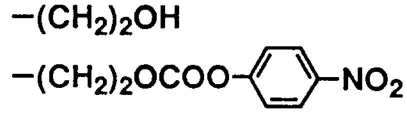 |
| 2 | c | 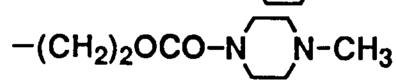 |
| 3 | d | $-COCH_2Cl$ |
| 3 | e | 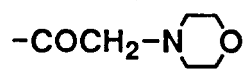 |
| 4 | f | $-COCH_2N(CH_3)_2$ |
| 5 | g | 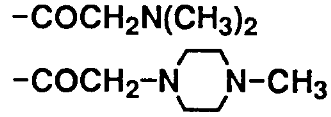 |
| 6 | h | 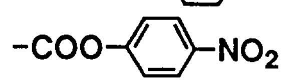 |
| 6 | i | 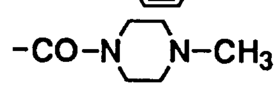 |
| 7 | j | $-COOCH_2CH_3$ |

Reference Example 1 Compound (I-8) and Compound (I-9)

In a mixture of 50 ml of tetrahydrofuran and 5 ml of water were dissolved 466 mg (1 mmol) of staurosporin and 360 mg (3 mmol) of glycolaldehyde dimer, and the solution was adjusted to pH 5 to 6 with a 3N aqueous solution of hydrochloric acid. To the solution was added 188 mg (3 mmol) of sodium cyanoborohydride at room temperature, followed by stirring for 2 hours. The reaction solution was washed with a saturated aqueous solution of sodium bicarbonate and dried over anhydrous sodium sulfate. The solvent was removed by distillation, and the residue was purified by silica gel column chromatography (5% methanol/chloroform) to give 460 mg (90%) of Compound a.

$^1$H-NMR (DMSO-$d_6$) δ (ppm); 4.99 (s, 2H), 6.86 (dd, 1H, J = 3.43, 8.87), 7.27-8.07 (m, 7H), 8.54 (s, 1H), 9.30 (d, 1H, J = 7.78)

SIMS (m/z); 511 $(M+1)^+$.

The resulting Compound a (300 mg) was dissolved in a mixture of 20 ml of dimethyl sulfoxide and 6.6 ml of a 0.5N aqueous solution of sodium hydroxide, and the solution was stirred at room temperature overnight. The reaction solution was diluted with tetrahydrofuran, washed with a saturated aqueous solution of sodium chloride, and dried over anhydrous sodium sulfate. The solvent was removed by distillation, and the residue was purified by silica gel column chromatography (5% methanol/chloroform) to give 140 mg

(45%) of an about 1:1 mixture of compound (I-9) and compound (I-10). The mixture was then subjected to high performance liquid chromatography (28% aqueous ammonia/water/methanol = 1/20/80) to give compound (I-8) and compound (I-9) as a single compound.

(I-8)

$^1$H-NMR (DMSO-$d_6$) δ (ppm); 2.149 (s, 3H), 2.403 (s, 3H), 4.253 (br.s, 1H), 6.436 (s, 2H), 6.844 (dd, 1H, J = 3.6, 8.9), 7.282-8.454 (m, 7H), 8.763 (s, 1H), 9.240 (dd, 1H, J = 1.0, 7.9)

SIMS (m/z); 527 $(M+1)^+$.

(I-9)

$^1$H-NMR (DMSO-$d_6$) δ (ppm); 2.202 (s, 3H), 2.397 (s, 3H), 2.803 (m, 1H), 4.256 (br.s, 1H), 6.429 (s, 2H), 6.844 (dd, 1H, J = 4.1, 9.0), 7.281-8.508 (m, 7H), 8.768 (s, 1H), 9.244 (dd, 1H, J = 1.0, 7.9)

SIMS (m/z); 527 $(M+1)^+$.

Reference Example 2 Compound (I-7)

In 25 ml of tetrahydrofuran were dissolved 484 mg (0.95 mmol) of Compound a obtained in Reference Example 1 and 382 mg (1.9 mmol) of p-nitrophenyl chloroformate, and 0.26 ml of triethylamine was added thereto under ice cooling, followed by stirring for 3 hours. The reaction solution was washed successively with a saturated aqueous solution of sodium bicarbonate and a saturated aqueous solution of sodium chloride, and dried over anhydrous sodium sulfate. The solvent was removed by distillation, and the residue was purified by silica gel column chromatography (10% acetone/chloroform) to give 394 mg (86%) of Compound b.

$^1$H-NMR (DMSO-$d_6$) δ (ppm); 2.12 (s, 3H), 2.42 (s, 3H), 2.61 (s, 3H), 3.95 (t, 1H, J = 5.4), 4.97 (s, 2H), 6.86 (dd, 1H, J = 3.4, 8.3), 6.92-8.30 (m, 11H), 8.53 (s, 1H), 9.30 (d, 1H, J = 8.0)

SIMS (m/z); 676 $(M+1)^+$.

In 15 ml of chloroform were dissolved 168 mg (0.26 mmol) of Compound b obtained above and 88 μl (0.79 mmol) of N-methylpiperazine, and 0.11 ml (0.79 mmol) of triethylamine was added thereto, followed by stirring overnight. The reaction solution was washed successively with a saturated aqueous solution of sodium bicarbonate, water, and a saturated aqueous solution of sodium chloride, and dried over anhydrous sodium sulfate. The solvent was removed, and the residue was purified by silica gel column chromatography (3% methanol/chloroform) to give 77 mg (46%) of Compound c.

$^1$H-NMR (DMSO-$d_6$) δ (ppm); 2.10 (s, 3H), 2.41 (s, 3H), 2.60 (s, 3H), 3.76 (m, 2H), 4.24 (s, 2H), 4.98 (s, 2H), 6.85 (dd, 1H, J = 3.42, 8.48), 7.27-8.05 (m, 7H), 8.53 (s, 1H), 9.30 (d, 1H, J = 7.84)

SIMS (m/z); 637 $(M+1)^+$.

In the same manner as in Reference Example 1, except for using 74 mg of Compound c obtained above in place of Compound a, 28 mg (37%) of Compound (I-7) was obtained.

$^1$H-NMR (DMSO-$d_6$) δ (ppm); 4.23 (s, 1H), 7.28-8.50 (m, 7H), 8.75 (s, 1H), 9.23 (d, 1H, J = 8.01)

SIMS (m/z); 653 $(M+1)^+$.

Reference Example 3 Compound (I-4)

In 10 ml of pyridine was dissolved 932 mg of staurosporin, and 0.64 ml of chloroacetyl chloride was added thereto under ice cooling, followed by stirring for 8 hours. The reaction solution was diluted with chloroform, washed successively with a 1N aqueous solution of hydrochloric acid, a saturated aqueous solution of sodium bicarbonate, and a saturated aqueous solution of sodium chloride, and dried over anhydrous sodium sulfate. The solvent was removed by distillation, and the residue was purified by silica gel column chromatography (10% methanol/chloroform) to give 184 mg (18%) of Compound d.

$^1$H-NMR (DMSO-$d_6$) δ (ppm); 5.01 (s, 2H), 7.03-7.08 (m, 1H), 7.28-8.29 (m, 7H), 9.31 (d, 1H, J = 8.06)

SIMS (m/z); 543 $(M+1)^+$.

In 20 ml of chloroform were dissolved 200 mg (0.37 mmol) of Compound d and 0.16 ml (1.85 mmol) of morpholine, and 0.26 ml (1.85 mmol) of triethylamine and 0.32 ml (1.85 mmol) of diisopropylethylamine were added thereto, followed by stirring for 36 hours. The reaction solution was washed with a saturated aqueous solution of sodium chloride, and dried over anhydrous sodium sulfate. The solvent was removed by distillation, and the residue was purified by silica gel column chromatography (5% methanol/ethyl acetate) to give 186 mg (85%) of Compound e.

$^1$H-NMR (DMSO-$d_6$) δ (ppm); 2.35 (s, 2.1H), 2.44 (s, 0.9H), 2.62 (s, 0.9H), 2.66 (s, 0.9H), 2.78 (s, 2.1H), 2.88 (s, 2.1H), 4.24, 4.49 (2xs, 2H), 5.00 (s, 2H), 7.00 (dd, 0.3H, J = 5.76, 7.83), 7.03 (dd, 0.7H, J = 6.74, 8.32), 7.28-8.08 (m, 7H), 8.55 (s, 1H), 9.28 (d, 0.7H, J = 7.74)

SIMS (m/z); 594 $(M+1)^+$.

In the same manner as in Reference Example 1, except for using 150 mg of Compound e obtained above in place of Compound a, 93 mg (61%) of Compound (I-4) was obtained.

$^1$H-NMR (DMSO-$d_6$) δ (ppm); 2.32, 2.33 (2xs, 3H), 2.88, 2.90 (2xs, 3H), 6.45 (s, 1H), 7.01-7.06 (m, 1H), 7.29-8.50 (m, 7H), 8.81 (s, 1H), 9.23, 9.25 (2xd, 1H, J = 7.90)

SIMS (m/z); 610 $(M+1)^+$.

Reference example 4 Compound (I-5)

In the same manner as in Reference Example 3, 190 mg (95%) of Compound f was obtained from 200 mg (0.37 mmol) of Compound d and 0.66 ml (7.37 mmol) of dimethylamine (50% aqueous solution).

$^1$H-NMR (DMSO-$d_6$) δ (ppm); 4.24, 4.38 (2xbr.s, 1H), 5.00 (s, 2H), 6.98 (dd, 0.31H, J = 5.12, 9.05), 7.03 (dd, 0.69H, J = 6.68, 8.48), 7.28-8.08 (m, 7H), 8.56 (s, 1H), 9.29 (d, 0.69H, J = 8.07)

SIMS (m/z); 552 $(M+1)^+$.

In the same manner as in Reference Example 1, except for using 180 mg of Compound f obtained above in place of Compound a, 104 mg (56%) of Compound (I-5) was obtained.

$^1$H-NMR (DMSO-$d_6$) δ (ppm); 4.24 (s, 0.33H), 4.27 (s, 0.33H), 4.39 (s, 0.17H), 4.42 (s, 0.17H), 6.44 (s, 0.17H), 6.46 (s, 0.33H), 7.30-8.52 (m, 7H), 8.78 (s, 1H), 9.22 (d, 0.66H, J = 8.27), 9.24 (d, 0.34H, J = 8.39)

SIMS (m/z); 568 $(M+1)^+$.

Reference Example 5 Compound (I-6)

In the same manner as in Reference Example 3, 181 mg (81%) of Compound g was obtained from 200 mg (0.37 mmol) of Compound d and 0.21 ml (1.85 mmol) of N-methylpiperazine.

$^1$H-NMR (DMSO-$d_6$) δ (ppm); 4.24 (m, 0.75H), 4.48 (m, 0.25H), 5.00 (s, 2H), 6.98 (dd, 0.25H, J = 5.66, 8.64), 7.03 (dd, 0.75H, J = 6.65, 8.42), 7.28-8.09 (m, 7H), 8.56, 8.57 (2xs, 1H), 9.28, 9.30 (2xd, 1H, J = 7.44)

SIMS (m/z); 607 $(M+1)^+$.

In the same manner as in Reference Example 1, except for using 162 mg of Compound g obtained above in place of Compound a, 99 mg (59%) of Compound (I-6) was obtained.

$^1$H-NMR (DMSO-$d_6$) δ (ppm); 4.24 (s, 0.35H), 4.26 (s, 0.3H), 4.48 (s, 0.2H), 4.51 (s, 0.15H), 7.30-8.52 (m, 7H), 8.78 (s, 0.65H), 8.79 (s, 0.35H), 9.22 (d, 0.65H, J = 7.56)

SIMS (m/z); 623 $(M+1)^+$.

Reference Example 6 Compound (I-3)

In 20 ml of chloroform were dissolved 233 mg of staurosporin and 301 mg of p-nitrophenyl chloroformate, and 0.21 ml (1.5 mmol) of triethylamine was added thereto under ice cooling, followed by stirring for 4 days. The reaction solution was washed successively with a saturated aqueous solution of sodium bicarbonate and a saturated aqueous solution of sodium chloride, and dried over anhydrous sodium sulfate. The solvent was removed by distillation, and the residue was purified by silica gel column chromatography (10% acetone/chloroform) to give 265 mg (84%) of Compound h.

$^1$H-NMR (DMSO-$d_6$) δ (ppm); 2.77 (s, 3H), 5.01 (s, 2H), 7.05 (m, 1H), 7.30-8.09 (m, 7H), 8.59 (s, 1H), 9.31 (d, 1H, J = 7.92)

SIMS (m/z); 632 $(M+1)^+$.

In 5 ml of dimethylformamide were dissolved 175 mg (0.27 mmol) of Compound h and 15 ml (1.4 mmol) of N-methylpiperazine, and 0.2 ml (1.4 mmol) of triethylamine was added thereto, followed by stirring at 70 °C for 5.5 hours. The reaction solution was diluted with chloroform, washed successively with a saturated aqueous solution of sodium bicarbonate, water, and a saturated aqueous solution of sodium chloride, and dried over anhydrous sodium sulfate. The solvent was removed by distillation, and the residue was purified by silica gel column chromatography (10% methanol/chloroform) to give 145 mg (90%) of Compound i.

$^1$H-NMR (DMSO-$d_6$) δ (ppm); 2.39 (s, 3H), 2.59 (s, 3H), 2.66 (s, 3H), 4.36 (d, 1H, J = 0.78), 4.42 (ddd, 1H, J = 2.26, 4.88, 12.87), 5.00 (s, 2H), 6.98 (dd, 1H, J = 5.22, 8.84), 7.28-8.07 (m, 7H), 8.56 (s, 1H), 9.29 (d, 1H, J = 7.91)

SIMS (m/z); 593 $(M+1)^+$.

In the same manner as in Reference Example 1, except for using 129 mg of Compound i obtained above in place of Compound a, 69 mg (49%) of Compound (I-3) was obtained.

$^1$H-NMR (DMSO-$d_6$) δ (ppm); 4.35 (s, 1H), 6.87 (s, 1H), 6.96 (dd, 1H, J = 5.02, 8.79), 7.29-8.51 (m, 7H), 8.78 (s, 1H), 9.23 (d, 1H, J = 7.94)

SIMS (m/z); 609 $(M+1)^+$.

Reference Example 7 Compound j

In a mixture of 27 ml of acetone and 18 ml of water were dissolved 932 mg (2 mmol) of staurosporin and 840 mg of sodium bicarbonate, and 0.29 ml of ethyl chloroformate was added thereto under ice cooling, followed by stirring for 1.5 hours. The reaction solution was diluted with chloroform, washed successively with water and an aqueous solution of sodium chloride, and dried over anhydrous sodium sulfate. The solvent was removed by distillation, and the residue was recrystallized from ethyl acetate to give 878 mg of Compound j.

$^1$H-NMR (DMSO-$d_6$) δ (ppm); 1.982 (s, 3H), 2.240 (m, 1H), 2.360 (s, 3H), 4.273 (br.s, 1H), 4.993 (s, 2H), 7.007 (dd, 1H, J = 6.2, 8.6), 7.274-8.065 (m, 7H), 8.551 (s, 1H), 9.281 (d, 1H, J = 8.0)

Industrial Applicability

According to the present invention there is provided an agent for potentiating sensitivity to an antitumor agent useful as a drug.

**Claims**

**1.** An agent for potentiating sensitivity to an antitumor agent comprising, as an active ingredient, an indolocarbazole derivative represented by formula (I):

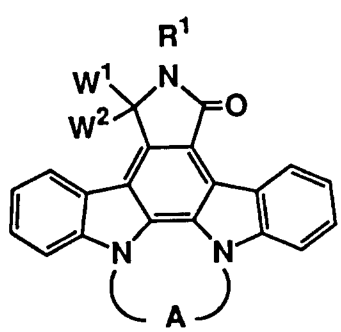

(I)

wherein

when $R^1$ represents hydrogen, lower alkyl, or lower alkanoyl, one of $W^1$ and $W^2$ represents hydrogen with the other representing hydrogen or hydroxy, or alternatively $W^1$ and $W^2$ are combined to represent oxygen, then A represents

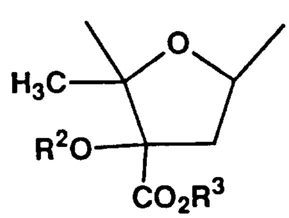

(wherein $R^2$ represents hydrogen, lower alkyl, or lower alkanoyl; and $R^3$ represents hydrogen or lower alkyl), with the proviso that when $R^1$, $R^2$, $W^1$, and $W^2$ are hydrogen, then $R^3$ is hydrogen or other lower alkyl than methyl; when $R^1$ represents lower alkyl or lower alkanoyl, one of $W^1$ and $W^2$ represents hydrogen with the other representing hydrogen or hydroxy, or when $R^1$ represents hydrogen, and one

of $W^1$ and $W^2$ represents hydrogen with the other representing hydroxy, then A represents

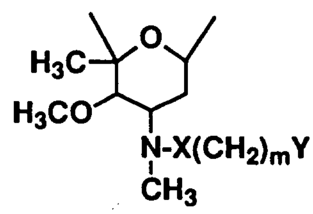

[wherein X represents a single bond or -CO-; m represents an integer of 0 to 6; and Y represents hydrogen, $OR^4$ (wherein $R^4$ represents hydrogen, lower alkyl, aralkyl, or $CONR^5R^6$, wherein $R^5$ and $R^6$ independently represent hydrogen or lower alkyl, or alternatively $R^5$ and $R^6$ are taken together with the nitrogen atom to form a heterocyclic group), $NR^7R^8$ (wherein $R^7$ and $R^8$ independently represent hydrogen or lower alkyl, or alternatively $R^7$ and $R^8$ are taken together with the nitrogen atom to form a heterocyclic group), or substituted or unsubstituted aryl;
or a pharmaceutically acceptable salt thereof.

**2.** A method for potentiating sensitivity to an antitumor agent comprising administration of an effective amount of an indolocarbazole derivative represented by formula (I) according to claim 1 or a pharmaceutically acceptable salt thereof.

**3.** The use of an indolocarbazole derivative represented by formula (I) according to claim 1 or a pharmaceutically acceptable salt thereof for the preparation of a pharmaceutical composition useful for potentiation of sensitivity to an antitumor agent.

**4.** An indolocarbazole derivative represented by formula (II):

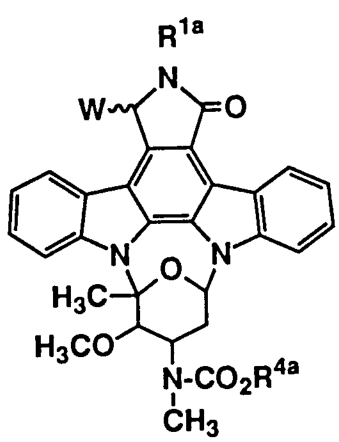

(II)

wherein $R^{1a}$ represents lower alkyl or lower alkanoyl; W represents hydrogen or hydroxy; and $R^{4a}$ represents lower alkyl;
or a pharmaceutically acceptable salt thereof.

## INTERNATIONAL SEARCH REPORT

International application No.
PCT/JP94/00275

A. CLASSIFICATION OF SUBJECT MATTER

Int. $Cl^5$ A61K31/475, C07D498/22

According to International Patent Classification (IPC) or to both national classification and IPC

B. FIELDS SEARCHED

Minimum documentation searched (classification system followed by classification symbols)

Int. $Cl^5$ A61K31/475, C07D498/22

Documentation searched other than minimum documentation to the extent that such documents are included in the fields searched

Electronic data base consulted during the international search (name of data base and, where practicable, search terms used)

CAS ONLINE

C. DOCUMENTS CONSIDERED TO BE RELEVANT

| Category* | Citation of document, with indication, where appropriate, of the relevant passages | Relevant to claim No. |
|---|---|---|
| A | JP, A, 61-268687 (Meiji Seika Kaisha, Ltd.), November 28, 1986 (28. 11. 86), (Family: none) | 1-4 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| April 19, 1994 (19. 04. 94) | May 17, 1994 (17. 05. 94) |
| Name and mailing address of the ISA/ Japanese Patent Office | Authorized officer |
| Facsimile No. | Telephone No. |